# EUROPEAN PATENT SPECIFICATION

(11) **EP 3 209 363 B1**
(45) Date of publication and mention of the grant of the patent: **27.03.2019**
(21) Application number: 15723570.6
(22) Date of filing: 27.03.2015
(51) Int. Cl.: A61M 25/06

(54) **INTRAVENOUS (IV) CATHETER WITH NEEDLE STICK SAFETY MECHANISM**
INTRAVENÖSER (IV) KATHETER MIT NADELSTICHSICHERHEITSMECHANISMUS
CATHÉTER INTRAVEINEUX (IV) COMPRENANT UN MÉCANISME DE SÉCURITÉ ANTI-PIQÛRE D'AIGUILLE

(30) Priority: 22.10.2014 IN 3031DE2014
(43) Date of publication of application: 30.08.2017
(73) Proprietor: Gupta, Neeraj, Gurgaon 122015 (IN)
(72) Inventor: Gupta, Neeraj, Gurgaon 122015 (IN)
(74) Representative: AWA Sweden AB
(86) International application number: PCT/IN2015/000147
(87) International publication number: WO 2016/063287

(56) References cited:
- US-A- 5 458 658
- US-A1- 2009 082 732

## Description

### FIELD OF THE INVENTION

The present subject matter relates to a medical apparatus, more preferably relates to an intravenous (IV) catheter introducing device with needle stick safety mechanism.

### BACKGROUND OF THE INVENTION

Over-needle catheters have been in existence for many decades and are now commonly used in hospitals all over the world. Though such catheters have seen many improvements in the past, a device that also provides protection to a user from a "needle stick" injuries is not yet so prevalent. The risks associated with such needle stick injuries are high, because of the high prevalence of communicable diseases among patients who reach hospitals and especially in emergency rooms of hospitals where cannulation treatment must be initiated immediately. The costs associated with any complication or adverse effects associated with such needle stick injuries are also very high.

US patent no. US 5,215,528 discloses a catheter introducer assembly including needle tip shield to protect user from a "needle stick" injuries. The catheter introducer assembly includes a needle hub having a needle secured thereto. The needle includes an elongated shaft having a bevelled tip. A portion of the needle shaft adjacent to the tip has a relatively large outside diameter. The shaft diameter of this portion exceeds the diameter, of the shaft portion adjoining the tip and the diameter of the shaft portion extending between the enlarged shaft portion and the needle hub. A catheter is mounted over the shaft of the needle, and includes an inner surface which bears against the enlarged shaft portion. A substantially leak-proof seal is thereby provided between the catheter and the needle shaft. A needle tip cover is slidably mounted to the needle shaft and is engageable with the enlarged portion of the shaft to prevent its removal therefrom. A sub-assembly of catheter and catheter hub is releasably mounted to the needle tip cover. However, to operate such catheter introducer assembly, a user requires additional training. Also, the needle catheter assembly is not robust as the user remains in contact with the needle tip cover and in case the needle cover tip is manipulated by the user during application, the user gets exposed to the needle tip and thus the risk of injuries to the user while operating the catheter introducer assembly is high. The manufacturing of such needle catheter assembly is complex and not cost effective.

See as well US5458658 A and US2009082732 A1.

### OBJECTS OF THE INVENTION

The main object of the present subject matter is to provide an intravenous (IV) catheter introducing device with needle stick safety mechanism.

Another object of the present subject matter is to provide robust construction of the IV catheter introducing device.

A further object of the present subject matter is to completely conceal the needle cover to prevent a user to come into contact with a "needle stick" which further prevents injuries to the user while operating the IV catheter introducing device.

It is yet another object of the present subject matter is to provide an IV catheter introducing device with needle stick safety mechanism which can be operated by the users without additional training.

It is further object of the present subject matter is to provide an IV catheter introducing device with needle stick safety mechanism which is reliable, easy and cost effective in manufacturing.

### SUMMARY OF THE INVENTION

Accordingly, the present invention, as claimed in claim 1, provides an intravenous (IV) catheter introducing device with a needle stick safety mechanism, the IV catheter introducing device comprises a needle having a first part, a second part and a third part. The third part of the needle has a diameter larger than the first part and the second part. A catheter tube slidably encases the third part and partially encases the first part and the second part of the needle. The IV catheter introducing device has a catheter hub which is in a fluid communication with the catheter tube and a flashback chamber. The IV catheter introducing device with needle stick safety mechanism comprises a needle cover assembly that partially encases the first part of the needle, and is connected to the catheter hub. The needle cover assembly comprises a needle cover having a safety element with blocking holes preventing the passing through of the third part of the needle. The needle cover is connected to the catheter hub by a lock and jaws and by engaging a needle cover hole with a projection on the catheter hub. The needle cover assembly further comprises a needle hub which has a through hole in which the needle is press fitted and the needle hub is connected to the flashback chamber through an extended portion. The needle cover assembly also comprises a casing enclosing both the needle cover and the needle hub, and the casing is connected to the needle cover via projections to prevent the rotation of the needle cover with the casing. The casing is also connected to the needle hub by engaging flanges and hooks in slots provided on the needle hub.

According to a preferred embodiment of the present subject matter, the safety element comprises a leaf spring connected to the needle cover via a recess, and a washer assembled with the needle cover via projections.

According to another preferred embodiment of the present subject matter, the blocking hole has diameter smaller than the third part of the needle.

According to yet another preferred embodiment of the present subject matter, the washer is a metallic washer.

According to still another preferred embodiment of the present subject matter, the washer is flat, conical or funnel shaped.

According to another preferred embodiment of the present subject matter, the needle cover hole and the projection on the catheter hub is formed to achieve a holding force which is higher than a sliding force of the needle and is lower than a holding force between the needle and the needle cover.

According to still another preferred embodiment of the present subject matter, the catheter hub is provided with wings.

According to yet another preferred embodiment of the present subject matter, the catheter hub is provided with a port.

According to another preferred embodiment of the present subject matter, the casing is provided with a thumb stop.

According to still another preferred embodiment of the present subject matter, the lock is a luer lock.

These and other features, aspects, and advantages of the present subject matter will become better understood with reference to the following description.

### BRIEF DESCRIPTION OF ACCOMPANYING DRAWINGS

The above and other features, aspects and advantages of the subject matter will be better understood with regard to the following description, and accompanying drawings. The figures show:
Fig. 1a shows a perspective view of an intravenous (IV) catheter introducing device in accordance with an embodiment of the present subject matter.
Fig. 1b shows a perspective view of the IV catheter introducing device in accordance with another embodiment of the present subject matter.
Fig. 1c shows a perspective view of the IV catheter introducing device in accordance with yet another embodiment of the present subject matter.
Fig. 2a, 2b, and 2c show perspective views of the needle cover.
Fig. 2d shows a perspective of a leaf spring.

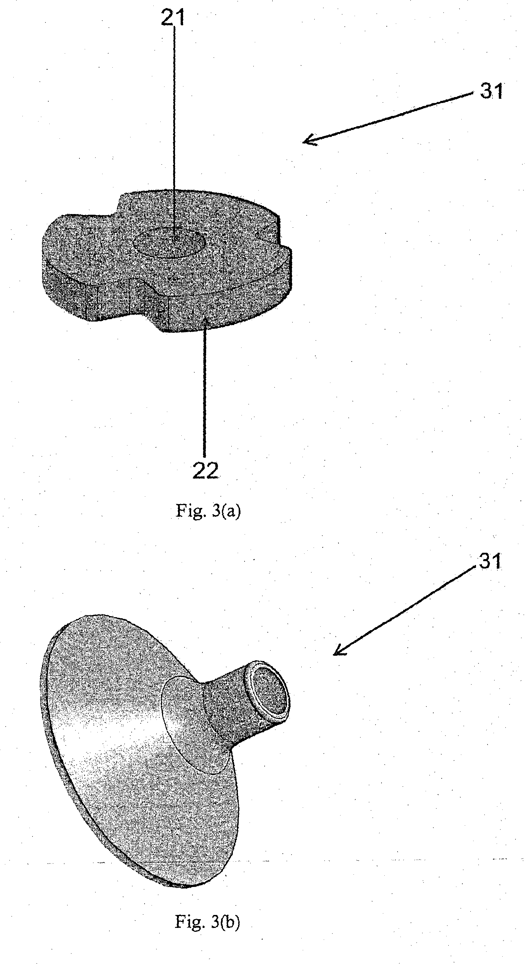

Fig. 3a shows a perspective view of a washer.
Fig. 3b shows an embodiment of a washer.
Fig. 4a shows a perspective view of a needle hub.
Fig. 4b shows a perspective view of a casing.
Fig. 4c shows a perspective view of the casing enclosing the needle cover.
Fig. 4d shows a perspective view of the casing enclosing the needle hub.

### DETAILED DESCRIPTION OF THE INVENTION

The present subject matter relates to an intravenous (IV) catheter introducing device with needle stick safety mechanism. In one implementation the IV catheter introducing device includes a needle having a first part, a second part and a third part. The third part of the needle has a diameter larger than the first part and the second part. A catheter tube slidably encases the third part and partially encases the first part and the second part of the needle. The IV catheter introducing device has a catheter hub which is in a fluid communication with the catheter tube and a flashback chamber. The IV catheter introducing device with needle stick safety mechanism comprises a needle cover assembly that partially encases the first part of the needle, and is connected to the catheter hub. The needle cover assembly comprises a needle cover having a safety element with a blocking hole preventing the passing through of the third part of the needle. The needle cover is connected to the catheter hub by a lock and jaws and by engaging a needle cover hole with a projection on the catheter hub. The needle cover assembly further comprises a needle hub which has a through hole in which the needle is press fitted. The needle hub is connected to the flashback chamber through an extended portion. The needle cover assembly also comprises a casing enclosing both the needle cover and the needle hub, and the casing is connected to the needle cover via projections to prevent the rotation of the needle cover with the casing. The casing is also connected to the needle hub by engaging flanges and hooks in slots provided on the needle hub.

According to a preferred embodiment of the present subject matter, the safety element comprises a leaf spring connected to the needle cover via a recess, and a washer assembled with the needle cover via projections.

The recess is provided on the needle cover in the direction opposite to the leaf spring. The leaf spring has two limbs in the direction towards the needle hub and a single leg in the direction towards the catheter hub. The width of the leg of the leaf spring is more than the width of the individual limbs. Moreover, a U-shaped channel is provided between the two limbs such that during withdrawal of the needle the enlarged portion on the circumference of the needle, i.e., the third part can pass through the U-shaped channel. The leg provided on the second end of the leaf spring stops the further forward movement of the needle and thereby avoids injuries to a patient or a user operating the IV catheter introducing device.

According to a preferred embodiment, the blocking hole has diameter smaller than the third part of the needle. The blocking hole is provided in the washer in a direction facing the flashback chamber. Further, the blocking hole has a diameter smaller in size than the size of the third part of the needle and thus the blocking hole prevents the passing of the needle through it. Accordingly, the blocking hole ensures that the needle after withdrawal from the IV catheter introducing device stays in the needle cover and therefore ensures that the user operating the IV catheter introducing device does not comes into contact with the needle. Thus the locking of needle in the needle cover prevents the injuries to the user operating the IV catheter introducing device and thereby ensuring the safety of the user.

According to a preferred embodiment, the washer is a metallic washer.

According to the preferred embodiment, the washer is flat, conical or funnel shaped.

According to a preferred embodiment, the needle cover hole and the projection on the catheter hub is designed to achieve a holding force which is higher than a sliding force of the needle and is lower than a holding force between the needle and the needle cover. The needle cover assembly provides an arrangement in which the needle cover is connected to the catheter hub by the lock and jaws provided on the needle cover and by engaging the needle cover hole with the projection on the catheter hub. The holding force between the needle cover and the catheter hub is higher than friction force which ensures that the needle cover cannot be withdrawn along with the needle during the withdrawal of the needle. Further, the holding force between the needle cover and catheter hub remains smaller than the force between the needle and the needle cover which ensures that the needle cover assembly is easily removed from the catheter hub during the withdrawal of the needle and which further ensures that the needle will remain in the needle cover during the withdrawal. The needle hub has a through hole in which the needle is press fitted and the needle hub is connected to the flashback chamber through an extended portion. The casing encloses both the needle cover and the needle hub. The casing is connected to the needle cover via the projections, which are provided on an end towards the needle hub, to prevent the rotation of the needle cover with the casing. The casing is also connected to the needle hub by engaging flanges and hooks in slots provided on the needle hub and thus ensures the safety of the user. Due to the above arrangement, during the withdrawal of the needle from the IV catheter introducing device, it is ensured that the needle is placed in the needle cover.

According to a preferred embodiment, the catheter hub is provided with wings. The wings are made of plastic and are provided for easy securement with the skin.

According to a preferred embodiment, the catheter hub is provided with a port. In an embodiment of the present subject matter, the port is provided on the catheter hub for conveying medicine or fluid to the patient through the catheter tube.

According to a preferred embodiment, the casing is provided with a thumb stop. The thumb stop is provided at an appropriate location so that the IV catheter introducing device can be held in a secure manner during its insertion into veins of the patient.

According to a preferred embodiment, the lock is a luer lock. The luer lock can be of an international standard 6% luer lock which is fitted into the catheter hub.

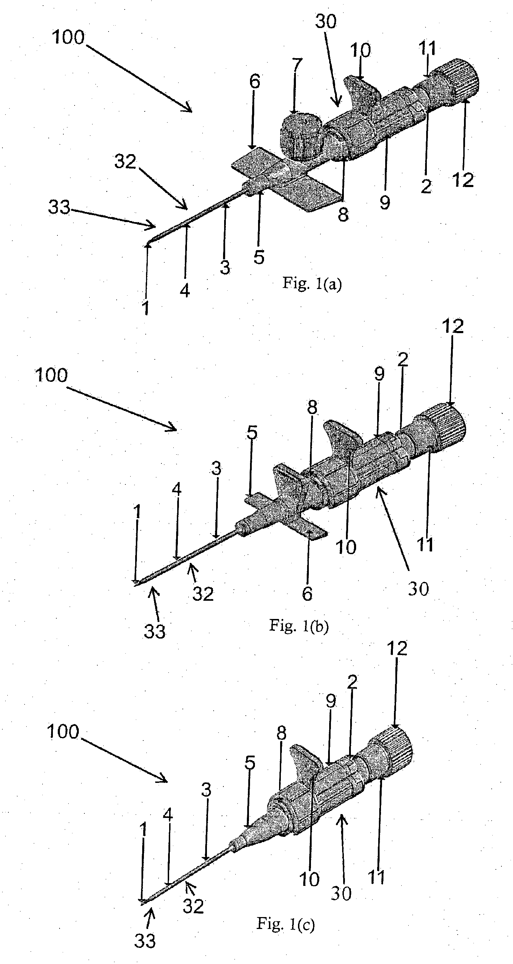

Figure 1a shows a perspective view of the IV catheter introducing device with a needle stick safety mechanism in accordance with an embodiment of the present subject matter. The IV catheter introducing device with a needle stick safety mechanism is indicated in Figure 1 by reference numeral 100.

The IV catheter introducing device 100 with needle stick safety mechanism includes a needle 1, a catheter tube 3 partially enclosing the needle 1, a catheter hub 5 which is connected to the catheter tube 3 in fluid communicating manner, a flashback chamber 11 and a needle cover sub assemble 8.

The needle 1 has three parts: a first part 32, a second part 33 and a third part 4 which has a diameter or a portion larger than the first part 32 and the second part 33. The third part 4 of the needle 1 has a lump on its circumference, i.e., the third part 4 has an enlarged portion on the circumference, which is preferably not all around 360 degrees but can be in a small part of the circumference of the needle 1. The third part 4 is slidably encased by the catheter tube 3. The third part 4 may be preferably designed on the needle 1 at a position between 15 to 25 mm from the bevel portion, which is at the tip of the needle 1, of the needle 1. The catheter tube 3 partially encases the first part 32 and the second part 33 of the needle 1. The first part 32 of the needle 1 passes through the catheter tube 3 to the needle cover assembly 8 via the catheter hub 5. The catheter hub 5, which may be made of plastic, is provided with wings 6 for easy securement with the skin. A port 7 is provided on the catheter hub 5 to supply medicine and other fluids to the patient via the catheter tube 3.

According to the preferred embodiment of the present subject matter, the catheter hub 5 is connected to the needle cover assembly 8 that partially encases the first part 32 of the needle 1. The needle cover assembly 8 comprises a casing 30, a needle cover 24 and a needle hub 2, where the casing 30 encloses or encases both the needle cover 24 and the needle hub 2 in a partial manner. The needle cover assembly 8 accommodates the first part 32 of the needle 1 such that it provides effective protection against needle stick injuries. The casing 30 receives the needle hub 2 in a fixed manner via projections 9 such that the needle cover 24 cannot be handled or manipulated during insertion of the catheter tube 3 in the vein or before the withdrawal of the needle 1 is performed. A thumb stop 10 may be provided on the casing 30 at a distance such that the IV catheter introducing device 100 can be held in a secure manner during insertion of the IV catheter introducing device 100 in the vein. The needle hub 2 is further connected to the flashback chamber 11 via an extended portion, where the flashback chamber 11 is provided to collect blood which confirms the venipuncture. The flashback chamber may be fitted with a hydrophobic filter which is provided to allow air to escape through it and allows blood to flow in. Further, a cap 12 may be provided at an end of the flashback chamber 11, where the cap 12 may be made of plastic.

Figure 1b shows another embodiment of the IV catheter introducing device 100 in perspective view. According to another preferred embodiment, the catheter hub 5 may be provided wings 6 which may have thumb stop to securely hold the IV catheter introducing device 100 during the insertion of the IV catheter introducing device 100 into the veins.

Figure 1c shows a perspective view of yet another embodiment of the present subject matter. The catheter hub 5 of the IV catheter introducing device may be directly connected to the needle cover assembly 8.

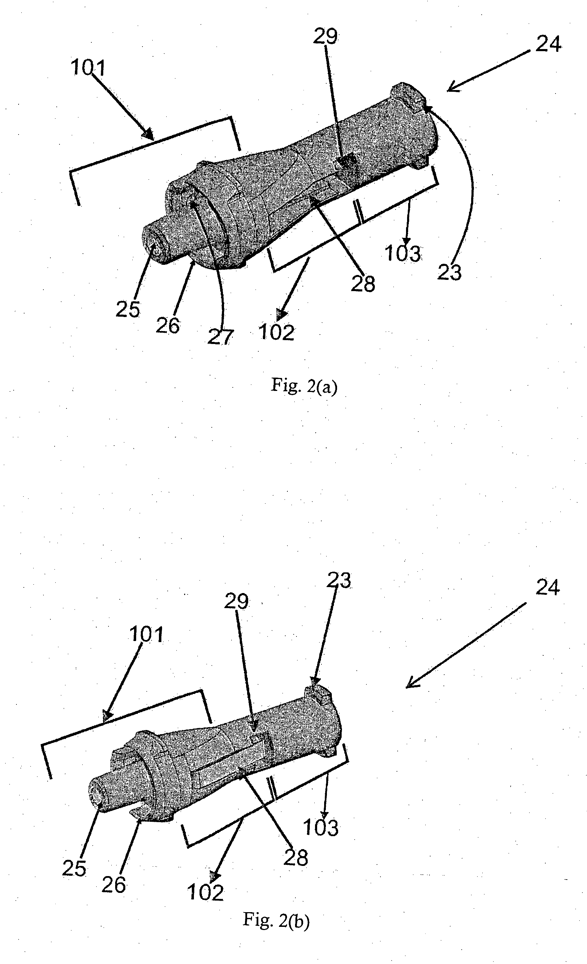

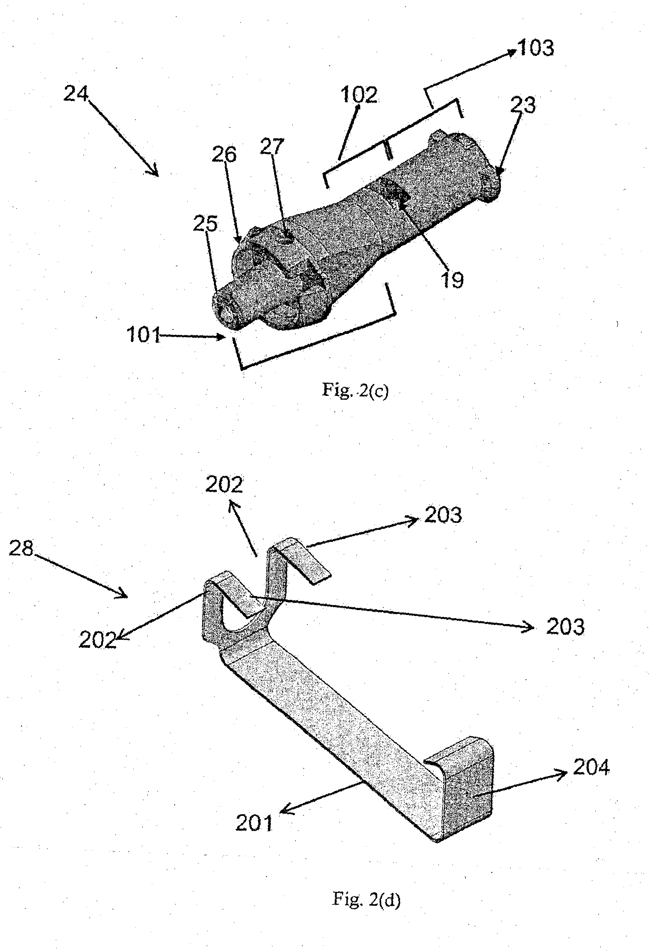

Figure 2a, 2b and 2c show perspective views of the needle cover 24 which accommodates the needle 1 after withdrawal from the IV catheter introducing device 100. The needle cover 24 includes three zones: first first zone01, second first zone02 and third first zone03.

The first zone 101 includes a lock 25, jaws 26 and a needle cover hole 27 which is a through hole. The first zone 101 of the needle cover 24 is connected to the catheter hub 5 via the lock 25 and jaws 26, where the lock 25 may be an international standard 6% luer lock. The first zone 101 may be further connected to the catheter hub 5 via locking the projections of the catheter hub 5 with the needle cover hole 27 (as shown in figure 2a and 2c) which is provided on the jaw 26 to provide a higher improved holding force to the connection of the catheter hub 5 and the needle cover 24 and thus a secure connection between the catheter hub 5 and the needle cover 24 is obtained. The size of the needle cover hole 27 and the corresponding projection on the catheter hub 5 can be modulated in order to achieve the desired holding force. The holding force thus achieved is higher than the sliding force of the needle 1 and is lower than the holding force between the needle 1 and the needle cover 24.

The second zone 102 and third zone 103 together forms a safety element which ensures that the tip of the needle cannot come into contact with the user. The second zone 102 includes a portion of the needle cover 24 that indicates a leaf spring arrangement. The leaf spring arrangement has a leaf spring .28 and a receiving slot 29 to accommodate the leaf spring 28. The figure 2d shows a perspective view of a leaf spring 28. The leaf spring has an elongated portion 201, two limbs 202 with a spring extended portion 203 on the side towards the needle hub 2 and a leg 204 on the side towards the catheter hub 5, where the width of the leg 204 of the leaf spring 28 is more than the individual limbs 202. The leaf spring 28 further defines a U-shaped channel between the limbs 202, which allow the passing of the needle through it during the withdrawal of the needle 1 by the user. Further, the leaf spring arrangement of the present disclosure allows insertion of the needle 1 through the U-shaped channel of the leaf spring 28 and thus enables a low assembly cost. Since, the U-shaped channel between the limbs 202 cannot stop the needle 1 passing through it therefore a common leaf spring can be used for any size of needle 1 which further ensures a low manufacturing cost of the leaf spring 28. The needle cover 24 include the recess 19 to securely receive the spring extended portion 203 in place in an engaging manner as shown in figure 2c. The leaf spring 28 is in two positions, initially, i.e., before the withdrawal of the needle 1, in an open position and during the withdrawal of the needle 1, it switches to the closed position.

The third zone 103 of the needle cover 24 includes a washer 31 which may be moulded integrally with the needle cover 24. The washer 31 may be a metallic washer. The washer 31 has a blocking hole 21 which prevents the passing of the needle 1 through it during the withdrawal operation of the needle 1 from the IV catheter introducing device 100. The blocking hole 21 of the washer 31 stops the third part 4 of the needle 1, which is wider on circumference than the blocking hole 21, from passing through it during the withdrawal of the needle 1 by the user. The blocking of the third part 4 of the needle 1 by the blocking hole 21 actuates the leaf spring 28 and switches the spring into closed position such that the leg 204 of the leaf spring 28 closes the through hole in the needle cover 24, which ensures that the tip at the second part 33 of the needle 1 is not allowed to move through the catheter hub 5 and the catheter tube 3 and thus ensures a safety of the patient and the user. The washer 31 may have projections 22 on its circumference, which helps in integral fixing of the washer 31 in the needle cover 24 in an easy manner. The washer 31 can be formed flat, conical or funnel shaped. The funnel shaped washer 31 ensures that the extent of enlargement of third part 4 on the circumference of the needle 1 can be reduced substantially and thus ensures that there is no interference between the third part 4 of the needle 1 and the catheter tube 3 and catheter hub 5. The third zone 103 further may have projections 23 at its circumference, which makes connection with the casing 30 in order to ensure that the needle cover 24 does not rotate within the casing 30 and thereby ensuring a robust design.

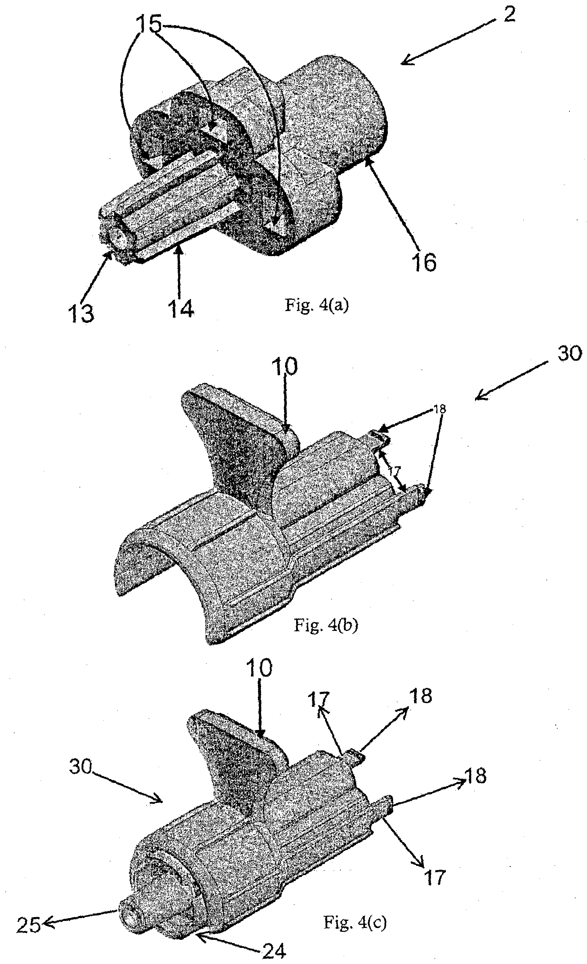

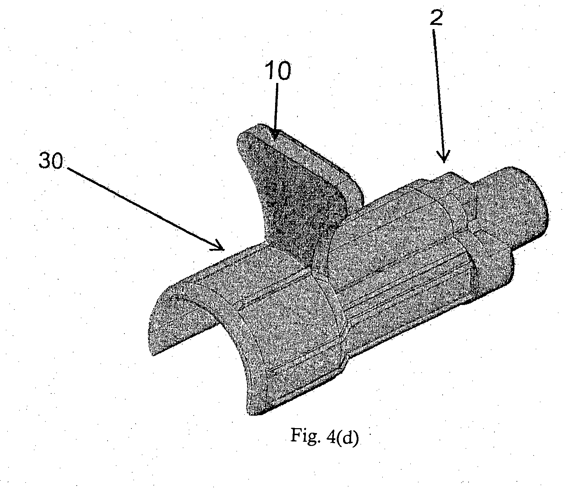

Figure 4a, 4b 4c and 4d show perspective views of the needle hub 2, casing 30, an assembly of the casing 30 and the needle cover 24, and an assembly of the casing 30 and the needle hub 2 respectively. The needle hub 2 includes a through hole 13 in which the needle 1 may be press fitted. The needle hub 2 is connected to the needle cover 24, preferably via a star shaped profile. The needle hub 2 has a plurality of slots 15 and an extended portion 16 connected to the flashback chamber 11. The casing 30 as shown in figure 4b may have a thumb 10 and mating flanges 17 which are provided with hooks 18. According to the preferred embodiment of present subject matter, the casing 30 makes an engaging connection with the needle hub 2 via inserting the flanges 17 into the slots 15 and locking the hooks 18 in the corresponding slots 15 as shown in figure 4d. As discussed above, the casing 30 encloses the needle cover 24 as shown in figure 4c. The aforementioned assembly enables that the needle 1 during the withdrawal cannot comes into contact with the user and remains into the needle cover assembly 8 as the holding force between the needle cover and the catheter hub can be precisely modulated such that the holding force can overcome friction force and ensures that it avoids the risk of dislodgement of the catheter tube. Further, the user does not come into direct contact with the needle as discussed in the above assembly arrangement and thus avoids the needle stick injuries caused to the user, which further prevents the risk of infection to the user.

Thus it is seen that the IV catheter introducing device with the needle stick safety mechanism is simple and easy to use, as the user does not require any additional training to operate the IV catheter introducing device.

### ADVANTAGES OF THE INVENTION

1. An intravenous (IV) catheter introducing device of the present subject matter provides a better protection to the user from the needle stick injuries that are caused during withdrawal operation of the needle from the veins.
2. The IV catheter introducing device of the present subject matter is easy and simple to use.
3. The user does not require any additional or special training to operate the IV catheter introducing device of the present subject matter unlike the catheter introducer assemblies known in the prior art.
4. The IV catheter introducing device of the present subject matter provides a protection to the patient and the user by stopping the further forward movement of the needle after the withdrawal operation.

Although the illustrative embodiments of the present subject matter have been described herein with reference to the accompanying drawings, it is to be understood that the present subject matter is not limited precisely to those embodiments, and that various other changes and modifications may be effected therein by person skilled in the art without departing from the scope of the present subject matter.

## Claims

1. An intravenous (IV) catheter introducing device (100) having needle stick safety mechanism comprising a needle (1), a catheter tube (3), a catheter hub (5), a needle cover assembly (8),
▪the needle (1) having a first part (32), a second part (33) and a third part (4) having a diameter larger than the first part (32) and the second part (33) of the needle (1);
the needle hub (2) having a through hole (13) in which the needle (1) is press fitted, the needle hub (2) being connected to the flashback chamber (11) through an extended portion (16),
▪the catheter tube (3) slidably encasing the third part (4) and partially encasing the first part (32) and the second part (33) of the needle (1);
the catheter hub (5) which is in a fluid communication with the catheter tube (3);
a flashback chamber (11); and
the needle cover assembly (8) partially encasing the first part (32) of the needle (1), and connected to the catheter hub (5),
▪the needle cover assembly (8) **characterized in that**
a needle cover (24) having a safety element with a blocking hole (21) preventing the passing through of the third part (4) of the needle (1), the needle cover (24) being connected to the catheter hub (5) by a lock (25) and jaws (26) and by engaging a needle cover hole (27) with a projection on the catheter hub (5), and
a casing (30) enclosing both the needle cover (24) and the needle hub (2), the casing (30) being connected to the needle cover (24) via projections (23) and to the needle hub (2) by engaging flanges (17) and hooks (18) in slots (15) provided on the needle hub (2).

2. The IV catheter introducing device (100) as claimed in claim 1, wherein the safety element comprises a leaf spring (28) connected to the needle cover (24) via a recess (19), and a washer (31) assembled with the needle cover (24) via projections (22).

3. The IV catheter introducing device (100) as claimed in claim 2, wherein the blocking hole (21) has diameter smaller than the third part (4) of the needle (1).

4. The IV catheter introducing device (100) as claimed in claim 2, wherein the washer (31) is a metallic washer.

5. The IV catheter introducing device (100) as claimed in claim 2, wherein the washer (31) is flat, conical or funnel shaped.

6. The IV catheter introducing device (100) as claimed in claim 1, wherein the needle cover hole (27) and the projection on the catheter hub (5) are formed to achieve a holding force which is higher than a sliding force of the needle (1) but lower than a holding force between the needle (1) and the needle cover (24).

7. The IV catheter introducing device (100) as claimed in claim 1, wherein the catheter hub (5) is provided with wings (6).

8. The IV catheter introducing device (100) as claimed in claim 1, wherein the catheter hub (5) is provided with a port (7).

9. The IV catheter introducing device (100) as claimed in claim 1, wherein the casing (30) is provided with a thumb stop (10).

10. The IV catheter introducing device (100) as claimed in claim 1, wherein the lock (25) is a luer lock.

## Patentansprüche

1. Vorrichtung (100) zum Einführen eines intravenösen (IV)-Katheters, die einen Nadelstichsicherheitsmechanismus hat, umfassend eine Nadel (1), eine Katheterröhre (3), einen Katheteransatz (5) und eine Nadelabdeckungsanordnung (8) umfasst,
wobei die Nadel (1) einen ersten Teil (32), einen zweiten Teil (33) und einen dritten Teil (4) mit einem Durchmesser hat, der größer als der erste Teil (32) und der zweite Teil (33) der Nadel (1) ist, wobei der Nadelansatz (2) ein Durchgangsloch (13) hat, in das die Nadel (1) pressgepasst ist, wobei der Nadelansatz (2) durch einen verlängerten Abschnitt (16) mit der Wasserverschlusskammer (11) verbunden ist,
wobei die Katheterröhre (3) den dritten Teil (4) der Nadel (1) gleitend und ihren ersten Teil (32) und ihren zweiten Teil (33) teilweise umschließt,
wobei der Katheteransatz (5) in Fluidverbindung mit der Katheterröhre (3) steht,
sowie eine Wasserverschlusskammer (11) und
wobei die Nadelabdeckungsanordnung (8) den ersten Teil (32) der Nadel (1) teilweise umschließt und mit dem Katheteransatz (5) verbunden ist,
wobei die Nadelabdeckungsanordnung (8) **dadurch gekennzeichnet ist, dass**
eine Nadelabdeckung (24) ein Sicherheitselement mit einem Blockierungsloch (21) hat, das den Durchgang des dritten Teils (4) der Nadel (1) verhindert, wobei die Nadelabdeckung (24) durch eine Verriegelung (25) und Backen (26) und dadurch mit dem Katheteransatz (5) verbunden ist, dass ein Nadelabdeckungsloch (27) mit einem Vorsprung am Katheteransatz (5) in Eingriff gebracht wird, und
ein Gehäuse (30) sowohl die Nadelabdeckung (24) als auch den Nadelansatz (2) umschließt, wobei das Gehäuse (30) über Vorsprünge (23) mit der Nadelabdeckung (24) verbunden ist und mit dem Nadelansatz (2), indem Flansche (17) und Haken (18) in am Nadelansatz (2) vorgesehenen Schlitzen (15) in Eingriff gebracht werden.

2. IV-Kathetereinführvorrichtung (100) nach Anspruch 1, wobei das Sicherheitselement eine Blattfeder (28), die über eine Aussparung (19) mit der Nadelabdeckung (24) verbunden ist, und eine Unterlegscheibe (31), die über Vorsprünge (22) mit der Nadelabdeckung (24) zusammengebaut ist, umfasst.

3. IV-Kathetereinführvorrichtung (100) nach Anspruch 2, wobei das Blockierloch (21) einen Durchmesser hat, der kleiner als der dritte Teil (4) der Nadel (1) ist.

4. IV-Kathetereinführvorrichtung (100) nach Anspruch 2, wobei die Unterlegscheibe (31) eine metallische Unterlegscheibe ist.

5. IV-Kathetereinführvorrichtung (100) nach Anspruch 2, wobei die Unterlegscheibe (31) flach, konisch oder trichterförmig ist.

6. IV-Kathetereinführvorrichtung (100) nach Anspruch 1, wobei das Nadelabdeckungsloch (27) und der Vorsprung am Katheteransatz (5) so ausgebildet sind, dass eine Haltekraft erzielt wird, die größer als eine Gleitkraft der Nadel (1), aber kleiner als eine Haltekraft zwischen der Nadel (1) und der Nadelabdeckung (24) ist.

7. IV-Kathetereinführvorrichtung (100) nach Anspruch 1, wobei der Katheteransatz (5) mit Flügeln (6) versehen ist.

8. IV-Kathetereinführvorrichtung (100) nach Anspruch 1, wobei der Katheteransatz (5) mit einem Port (7) versehen ist.

9. IV-Kathetereinführvorrichtung (100) nach Anspruch 1, wobei das Gehäuse (30) mit einem Daumenanschlag (10) versehen ist.

10. IV-Kathetereinführvorrichtung (100) nach Anspruch 1, wobei die Verriegelung (25) ein Luer-Lock ist.

## Revendications

1. Dispositif d'introduction (100) pour un cathéter intraveineux (IV) ayant un mécanisme de sécurité anti piqûre d'aiguille, comprenant une aiguille (1), un tube de cathéter (3), un moyeu de cathéter (5), un ensemble couvre-aiguille (8),
l'aiguille (1) ayant une première partie (32), une deuxième partie (33) et une troisième partie (4) ayant un diamètre plus grand que la première partie (32) et la deuxième partie (33) de l'aiguille (1) ; le moyeu d'aiguille (2) ayant un trou traversant (13) dans lequel l'aiguille (1) est ajustée par pressage, le moyeu d'aiguille (2) étant connecté à la chambre de retour (11) par une portion prolongée (16),
le tube de cathéter (3) enveloppant de manière coulissante la troisième partie (4) et enveloppant partiellement la première partie (32) et la deuxième partie (33) de l'aiguille (1) ;
le moyeu de cathéter (5) étant en communication fluidique avec le tube de cathéter (3) ;
ainsi qu'une chambre de retour (11) ; et
l'ensemble couvre-aiguille (8) enveloppant partiellement la première partie (32) de l'aiguille (1), et étant connecté au moyeu de cathéter (5),
l'ensemble couvre-aiguille (8) étant **caractérisé en ce que**
un couvre-aiguille (24) ayant un élément de sécurité avec un trou de blocage (21) empêchant le passage de la troisième partie (4) de l'aiguille (1), le couvre-aiguille (24) étant connecté au moyeu de cathéter (5) par un verrou (25) et des mâchoires (26) et par l'engagement d'un trou du couvre-aiguille (27) avec une saillie du moyeu de cathéter (5), et
un boîtier (30) renfermant à la fois le couvre-aiguille (24) et le moyeu d'aiguille (2), le boîtier (30) étant connecté au couvre-aiguille (24) par le biais de saillies (23) et au moyeu d'aiguille (2) par des brides d'engagement (17) et des crochets (18) dans des fentes (15) prévues sur le moyeu d'aiguille (2).

2. Dispositif d'introduction (100) pour un cathéter intraveineux (IV) selon la revendication 1, dans lequel l'élément de sécurité comprend un ressort à lame (28) connecté au couvre-aiguille (24) par le biais d'un renfoncement (19), et une rondelle (31) assemblée au couvre-aiguille (24) par le biais de saillies (22).

3. Dispositif d'introduction (100) pour un cathéter intraveineux (IV) selon la revendication 2, dans lequel le trou de blocage (25) présente un diamètre inférieur à la troisième partie (4) de l'aiguille (1) .

4. Dispositif d'introduction (100) pour un cathéter intraveineux (IV) selon la revendication 2, dans lequel la rondelle (31) est une rondelle métallique.

5. Dispositif d'introduction (100) pour un cathéter intraveineux (IV) selon la revendication 2, dans lequel la rondelle (31) est plate, conique ou en forme d'entonnoir.

6. Dispositif d'introduction (100) pour un cathéter intraveineux (IV) selon la revendication 1, dans lequel le trou du couvre-aiguille (27) et la saillie sur le moyeu de cathéter (5) sont formés de manière à réaliser une force de retenue qui est supérieure à une force de coulissement de l'aiguille (1) mais qui est inférieure à une force de retenue entre l'aiguille (1) et le couvre-aiguille (24).

7. Dispositif d'introduction (100) pour un cathéter intraveineux (IV) selon la revendication 1, dans lequel le moyeu de cathéter (5) est pourvu d'ailes (6) .

8. Dispositif d'introduction (100) pour un cathéter intraveineux (IV) selon la revendication 1, dans lequel le moyeu de cathéter (5) est pourvu d'un port (7) .

9. Dispositif d'introduction (100) pour un cathéter intraveineux (IV) selon la revendication 1, dans lequel le boîtier (30) est pourvu d'une butée pour le pouce (10).

10. Dispositif d'introduction (100) pour un cathéter intraveineux (IV) selon la revendication 1, dans lequel le verrou (25) est un verrouillage de Luer.
